# EUROPÄISCHE PATENTSCHRIFT

(11) **EP 1 942 834 B1**
(45) Veröffentlichungstag und Bekanntmachung des Hinweises auf die Patenterteilung: **16.01.2013**
(21) Anmeldenummer: 06806665.3
(22) Anmeldetag: 02.11.2006
(51) Int. Cl.: A61F 2/24

(54) **SELBSTEXPANDIERBARES MEDIZINISCHES INSTRUMENT ZUR BEHANDLUNG VON DEFEKTEN AM HERZEN EINES PATIENTEN**
SELF-EXPANDABLE MEDICAL INSTRUMENT FOR TREATMENT OF DEFECTS ON A PATIENT S HEART
INSTRUMENT MÉDICAL AUTO-EXTENSIBLE POUR LE TRAITEMENT DE DÉFAILLANCES CARDIAQUES CHEZ UN PATIENT

(30) Priorität: 04.11.2005 DE 102005052628
(43) Veröffentlichungstag der Anmeldung: 16.07.2008
(73) Patentinhaber: JenaValve Technology Inc., Wilmington, DE 19801 (US)
(72) Erfinder: FIGULLA, Hans-Reiner, 07749 Jena (DE); FERRARI, Markus, 07747 Jena (DE)
(74) Vertreter: Trinks, Ole
(86) Internationale Anmeldenummer: PCT/EP2006/010519
(87) Internationale Veröffentlichungsnummer: WO 2007/051620

(56) Entgegenhaltungen:
- WO-A2-2004/064671
- FR-A- 2 788 217
- FR-A1- 2 815 844
- US-A1- 2002 143 387
- US-A1- 2005 125 075
- US-A1- 2005 137 688
- US-B1- 6 241 738

## Beschreibung

Die vorliegende Erfindung betrifft ein selbstexpandierbares medizinisches Instrument zur Behandlung von Defekten am Herzen eines Patienten, insbesondere zur transvaskulären Implantation einer Herzklappenprothese, wobei das medizinische Instrument mittels eines Kathetersystems minimal-invasiv in den Körper eines Patienten einführbar ist. Insbesondere betrifft die Erfindung eine Vorrichtung zum transvaskulären Ersatz von erkrankten Herzklappen.

Eine derartige Vorrichtung ist dem Grunde nach aus der Medizintechnik bekannt. Gegenwärtig stehen für den Ersatz menschlicher Herzklappen biologische oder mechanische Klappenmodelle zur Verfügung, die in der Regel über eine Öffnung des Brustkorbs chirurgisch nach Entfernung der erkrankten Herzklappe im Herzklappenbett festgenäht werden. Für diesen Eingriff muss der Kreislauf des Patienten durch eine Herz-Lungen-Maschine getragen werden, wobei während der Implantation der Herzklappenprothese ein Herzstillstand induziert wird. Dabei handelt es sich um einen risikoreichen chirurgischen Eingriff mit entsprechenden Gefahren für den Patienten und mit einer langen postoperativen Behandlungsphase. Insbesondere kann ein solcher Eingriff bei multimorbiden Patienten oft nicht mehr mit einem vertretbaren Risiko durchgeführt werden.

In jüngster Zeit wurden minimal-invasive Therapieverfahren entwickelt, die sich insbesondere dadurch auszeichnen, dass der Eingriff in örtlicher Betäubung durchgeführt werden kann. Ein Ansatz sieht dabei vor, einen selbstexpandierenden Stent verbundenen mit einer zusammenfaltbaren Herzklappe über ein entsprechendes Kathetersystem im menschlichen Körper zu implantieren. Eine solche selbstexpandierende Herzklappenprothese kann mit Hilfe eines Kathetersystems durch eine Leistenarterie oder -vene bis hin zum Implantationsort am Herzen geführt werden. Nach Erreichen des Implantationsortes kann dann der Stent, der in seiner Längsrichtung beispielsweise aus mehreren relativ zueinander abwinkelbaren selbstexpandierenden Stent-Segmenten zusammengesetzt ist, sukzessiv entfaltet werden. Nach der Entfaltung kann die Herzklappenprothese beispielsweise mit Unterstützung von Verankerungshaken zumindest im herznahen Bereich im jeweiligen Blutgefäß verankert werden. Die eigentliche Herzklappenprothese befindet sich dabei unmittelbar im proximalen Bereich des Stents.

Beispielsweise ist aus der Druckschrift DE 100 10 074 A1 eine Vorrichtung zur Befestigung und Verankerung von Herzklappenprothesen bekannt, die im wesentlichen aus drahtförmigen, miteinander verbundenen Elementen gebildet ist. Dabei ist vorgesehen, dass unterschiedliche Bügel eingesetzt werden, um eine sichere Befestigung und Abstützung der Herzklappenprothese zu erreichen. Die in dieser Druckschrift beschriebene Vorrichtung verwendet dazu drei jeweils gleiche Paare von Bügeln, die in jeweils einem Abstand von 120° zueinander angeordnet sind. Diese Bügel sind miteinander durch Festkörpergelenke verbunden, wobei die Festkörpergelenke die Funktion von Drehlagern erfüllen. Zusätzlich sind entgegengesetzt gebogene Bügel vorhanden, die möglichst gleichlange Hebelarme bilden, um eine sichere Anlage der Bügel auch bei peristaltischen Bewegungen am Herzen und Blutgefäß und eine sichere Abdichtung einer implantierten und fixierten Herzklappenprothese erreichen zu können.

Bei den bekannten Lösungen besteht jedoch die Gefahr einer Fehlimplantation von Herzklappen. Dies betrifft im wesentlichen die exakte Positionierung und longitudinal Ausrichtung von der zu implantierenden Herzklappenprothese. Insbesondere ist es - wenn überhaupt - nur mit großem Geschick des behandelnden Chirurgen möglich, einen Stent, der in seinem proximalen Ende die Herzklappenprothese aufweist, so genau in der Nähe der kranken Herzklappe des Patienten zu positionieren, dass nicht nur eine hinreichende laterale Positionsgenauigkeit, sondern auch eine geeignete longitudinale Lage der Herzklappenprothese optimal sichergestellt werden können.

Eine Fehlimplantation einer beispielsweise nicht optimal positionierten Herzklappenprothese kann unter anderem zu einer Undichtigkeit bzw. Klappeninsuffizienz führen, was erhebliche Belastungen des Ventrikels nach sich zieht. Erfolgt beispielsweise eine Implantation einer Herzklappenprothese zu weit oberhalb der eigentlichen Herzklappenebene, kann es zum Verschluss der Abgänge der Herzkranzgefäße (Koronarien) und damit zu einer tödlichen Koronarischämie mit Herzinfarkt kommen. Demnach ist es zwingend erforderlich, dass sowohl die laterale Positionsgenauigkeit als auch die longitudinale Lage einer implantierten Herzklappenprothese den Erfordernissen entspricht.

Bei herkömmlichen Implantationstechniken, bei denen selbstexpandierbare Herzklappenprothesen durch beispielsweise eine Leistenarterie des Patienten minimal-invasiv bis an den Implantationsort am Herzen geführt werden, erfolgt die Einführung der Prothese üblicherweise mittels eines Führungsdrahtes und mit Hilfe von Kathetern, wobei auch herkömmliche Ballonkatheter eingesetzt werden können. Obwohl bei einem solchen Eingriff das Einführen, etwa mit Hilfe einer Röntgendurchleuchtung (Herzkatheterlabor = HKL) oder mit Hilfe von Ultraschall (transösophageales Echokardiogramm = TEE), überwacht und gesteuert werden kann, ist es - infolge der eingeschränkten Manövrierfähigkeit der beim Einführvorgang noch zusammengefalteten Herzklappenprothese, die trotz ihres zusammengefalteten Zustandes relativ große Dimensionen aufweist, häufig nicht möglich, die erforderlich Positionsgenauigkeit und insbesondere die longitudinale Lage der zu implantierenden Herzklappenprothese mit den entsprechend daran befestigten Befestigungselementen sicherzustellen. Insbesondere kann - infolge eines möglichen Verschlusses von Herzkranzgefäßen - ein Winkelversatz der implantierten Herzklappenprothese vom optimalen Implantationsort eine Gefährdung für den jeweiligen Patienten darstellen.

Bei der Auslegung einer Herzklappenprothese sind insbesondere auch die während der Füllungsphase des Herzzyklus (Diastole) an der Prothese wirkende, erhebliche Kräfte zu berücksichtigen, wonach eine sichere Verankerung erforderlich ist, um ein Ablösen der implantierten Herzklappenprothese zu verhindern.

Von daher muss sich einerseits die Herzklappenprothese bei dem Implantationsvorgang möglichst gut im entsprechenden Zugangsgefäß manövrieren lassen, um somit eine optimale Positionsgenauigkeit sicherzustellen, und andererseits muss sich die implantierte Prothese in ihrem Implantationsort fest verankern lassen, um einen nachträglichen Versatz der Prothese wirksam verhindern zu können.

Der vorliegenden Erfindung liegt die Problemstellung zugrunde, dass sich bekannte Vorrichtungen zur transvaskulären Implantation und Befestigung von Herzklappenprothesen häufig nicht eignen, auf einfache Weise eine Herzklappenprothese mit der notwendigen Positioniergenauigkeit zu implantieren. Darüber hinaus ist es bisher, wenn überhaupt, nur mit hohem Aufwand möglich, eine bereits implantite Herzklappenprothese mit einem minimal-invasiven Eingriff zu explantieren oder eine fehlerhaft positionierte Herzklappenprothese entsprechend zu korrigieren.

Ausgehend von dieser Problemstellung liegt der Erfindung nun als eine Aufgabe zugrunde, eine Vorrichtung zur transvaskulären Implantation und Befestigung von Herzklappenprothesen anzugeben, bei welcher die zuvor diskutierten Nachteile der herkömmlichen Implantationssysteme behoben sind.

Erfindungsgemäß wird diese Aufgabe mit einem medizinischen selbstexpandierbaren Instrument zur Behandlung von Defekten am Herzen eines Patienten, insbesondere zur transvaskulären Implantation einer Herzklappenprothese gelöst, wobei das medizinische Instrument mittels eines Kathetersystems minimal-invasiv in den Körper eines Patienten einführbar ist und einen Stent aus einem flexiblen Geflecht dünner Drähte oder Fäden besteht. Dabei ist vorgesehen, dass der Stent bzw. das Geflecht während des Einführens des medizinischen Instruments in den Körper des Patienten eine erste vorab festlegbare Formgebung und im implantierten Zustand des medizinischen Instruments eine zweite vorab festlegbare Formgebung aufweist, wobei das medizinische Instrument in der ersten Formgebung des Stents bzw. Geflechts in einem zusammengefalteten Zustand und in der zweiten Formgebung des Stents bzw. Geflechts in einem expandierten Zustand vorliegt. Insbesondere weist das medizinische Instrument nach der erfindungsgemäßen Lösung in seinem expandierten Zustand einen distalen Retentionsbereich mit einem lateral nach außen gestülpten Wulstabschnitt, welcher im implantierten Zustand des medizinischen Instruments mit zumindest einer Tasche der defekten Herzklappe des Patienten in Eingriff bringbar ist, einen proximalen Retentionsbereich sowie einen zwischen dem distalen und proximalen Retentionsbereich liegenden Mittenbereich auf. In seinem expandierten Zustand weist dabei das medizinische Instrument am Mittenbereich einen kleineren Durchmesser als am proximalen und/oder distalen Retentionsbereich auf, wobei der Mittenbereich ausgelegt ist, im implantierten Zustand des medizinischen Instruments auf Höhe der defekten Herzklappe des Patienten eine formschlüssige Verbindung mit der Gefäßwand an bzw. in unmittelbarer Nähe der defekten Herzklappe zu bilden.

Die Vorteile der Erfindung liegen insbesondere darin, dass ein transvaskulär einführbares medizinisches Instrument, insbesondere zur Behandlung von Defekten am Herzen eines Patienten, angegeben wird, wobei sich das medizinische Instrument für eine Zufuhr über einen Katheter zu dem zu behandelnden Defekt am Herzen des Patienten eignet. Dadurch, dass das medizinische Instrument als selbstexpandierbares Instrument ausgeführt ist und im wesentlichen einen Stent aus einem flexiblen Geflecht dünner Drähte bzw. Fäden aufweist, kann in besonders vorteilhafter Weise erreicht werden, dass sich das medizinische Instrument - unabhängig von der Größe der zu behandelnden Herzklappe und unabhängig von dem Durchmesser der defekten Herzklappe - an der defekten Herzklappe selbstständig anpasst, und zwar derart, dass zum einen die Anteile des medizinischen Instruments möglichst wenig in den an dem implantierten medizinischen Instrument vorbeiströmenden Blutstrom hineinragt, wobei gleichzeitig eine optimale Positionierung, sichere Verankerung und optimale seitliche Abdichtung des implantierten medizinischen Instruments gewährleistet werden.

Demnach kann erreicht werden, dass das medizinische Instrument optimal an der defekten Herzklappe positionierbar und dort äußerst stabil verankert werden kann, wobei gleichzeitig emboliebedingte Probleme verhindert werden können. Aus der Verwendung eines aus dünnen Drähten oder Fäden aufgebauten Geflechts aus Ausgangsmaterial für den Stent bzw. für das erfindungsgemäße medizinische Instrument leitet sich der weitere Vorteil ab, dass das medizinische Instrument eine langfristige mechanische Stabilität aufweist. Somit kann das Auftreten von Brüchen in der Struktur des eingesetzten Instruments dauerhaft verhindert werden. Ferner besitzt das Geflecht eine ausreichende Steifigkeit.

Kurz zusammengefasst zeichnet sich die erfindungsgemäße Lösung dadurch aus, dass das medizinische Instrument einen Stent aus einem flexiblen Geflecht, insbesondere Drahtgeflecht, aufweist, welcher sich beim Freisetzen aus dem Katheter pilzförmig in die Taschen der erkrankten Herzklappe hineinstülpt und diese durch Umschlagen einklemmt. Dadurch kann eine optimale Positionierung und stabile Verankerung einer sich in der Mitte Stents befindlichen bzw. vorgesehenen Herzklappenprothese erfolgen. Gleichzeitig wird eine optimale seitliche Abdichtung der implantierten Klappenprothese gewährleistet.

Bevorzugte Weiterbildungen des medizinischen Instruments sind in den Unteransprüchen angegeben.

So ist in einer besonders bevorzugten Realisierung des erfindungsgemäßen medizinischen Instruments vorgesehen, dass der Stent ferner eine im Mittenbereich angeordnete selbst-expandierbare Herzklappenprothese aufweist, die sich bei der Freisetzung des medizinischen Instruments aus dem Kathetersystem selbstständig entfaltet und dann die Funktion der defekten Herzklappe des Patienten übernimmt. Bei dieser bevorzugten Ausführungsform dient somit das Geflecht des medizinischen Instruments als Herzklappenstent zum Verankern und Positionieren der im Mittenbereich des medizinischen Instruments angeordneten Herzklappenprothese. Insbesondere zeichnet sich das medizinische Instrument dadurch aus, dass es aufgrund seiner Formgebung im expandierten Zustand nicht nur eine äußerst stabile Verankerung der Herzklappenprothese, sondern auch eine selbstständige Positionierung dieser in der Höhe der zu ersetzenden defekten Herzklappe bietet.

Hinsichtlich des Geflechts, welches den Herzklappenprothesenstent bildet, ist bevorzugt vorgesehen, dass dieses ein Schlauchgeflecht ist, so dass das medizinische Instrument in seinem expandierten Zustand eine zum proximalen und distalen Ende hin offene Form aufweist. Ein Schlauchgeflecht hat den Vorteil, dass im implantierten Zustand des expandierten medizinischen Instruments der Blutstrom durch das medizinische Instrument hindurchfließt, wobei - bis auf die im Mittenbereich des medizinischen Instruments vorgesehene Herzklappenprothese - nahezu keine Fremdkomponenten in den Blutstrom ragen.

Des weiteren ist es denkbar, dass bei dem expandierten medizinischen Instrument der Wulstabschnitt am distalen Retentionsbereich des Stents durch pilzförmiges Umstülpen des distalen Endes des Geflechts nach außen gebildet wird. Insbesondere ist dabei der Wulstabschnitt am distalen Retentionsbereich des expandierten medizinischen Instruments im implantierten Zustand des medizinischen Instruments in die zumindest eine Tasche der defekten Herzklappe des Patienten stülpbar und dient somit als ein sich selbständig positionierendes Mittel zum Positionieren des medizinischen Instruments auf Höhe der defekten Herzklappe des Patienten.

Nach einem weiteren Aspekt der vorliegenden Erfindung bildet bei dem expandierten medizinischen Instrument der proximale Retentionsbereich des Stents aufgrund der selbstexpandierbaren Eigenschaft des aus dem flexiblen Geflecht bestehenden Stents eine kraftschlüssige Verbindung mit der Gefäßwand und sorgt von daher für eine stabile Verankerung des implantierten medizinischen Instruments.

Andererseits ist in vorteilhafter Weise bei dem expandierten medizinischen Instrument der Mittenbereich des Stents ausgelegt, dass im implantierten Zustand des medizinischen Instruments aufgrund der selbstexpandierbaren Eigenschaft des aus dem flexiblen Geflecht gebildeten Stents der Mittenbereich die defekte Herzklappe des Patienten gegen die im Hinblick auf die defekte Herzklappe distal gelegene Gefäßwand drückt.

Hinsichtlich der Formgebung des medizinischen Instruments in seinem expandierten Zustand bzw. des Stents in seiner zweiten Formgebung ist bevorzugt, dass diese hantelähnlich ist, wobei sowohl der distale als auch proximale Retentionsbereich jeweils in Gestalt einer Pilzkappe ausgebildet sind. Des weiteren ist es bevorzugt; dass bei dem expandierten medizinischen Instrument der Mittenbereich des Stents einen im Vergleich zum proximalen und distalen Retentionsbereich kleineren Durchmesser aufweist, wobei der Mittenbereich eine Länge aufweist, die in etwa der Länge der defekten Herzklappe entspricht.

Insbesondere ist bevorzugt, dass sich der aus dem Geflecht gebildete Stent mit einer im Mittenbereich angeordneten selbst-expandierbaren Herzklappenprothese auf den Durchmesser eines bei dem transvaskulären Operationseingriff verwendeten Kathetersystems verjüngen lässt.

Um zu erreichen, dass das im Körper des Patienten bereits implantierte medizinische Instrument nachträglich wieder explantierbar ist, ist bei einer bevorzugten Weiterentwicklung der erfindungsgemäßen Lösung vorgesehen, dass der Stent an seinem proximalen und/oder distalen Ende eine mit einem Explantations-Kathetersystem in Eingriff bringbare Fassung aufweist, wobei ferner das medizinische Instrument ausgelegt ist, dass es sich durch externe Manipulation aus seinem expandierten Zustand in seinen zusammengefalteten Zustand bringen lässt, so dass das medizinische Instrument bzw. der Stent mit der Herzklappenprothese möglichst einfach explantiert werden kann.

Gemäß einem weiteren Aspekt der Erfindung ist vorgesehen, dass das flexible Geflecht, welches den Stent für das medizinische selbstexpandierbare Instrument bildet, im implantierten Zustand der medizinischen Vorrichtung in mehrschichtiger Anordnung um die defekte Herzklappe des Patienten herum liegt.

Durch die Verwendung des aus dem flexiblen Geflecht gebildeten Stents und aufgrund der damit erreichten selbstexpandierbaren Eigenschaft des medizinischen Instruments ist besonders bevorzugt vorgesehen, dass das der Stent mit der im Mittenbereich angeordneten selbst-expandierbaren Herzklappenprothese ausgelegt ist, dass die zweite Formgebung des Stents und somit das medizinische Instrument an den anatomischen Bedingungen so anpassbar ist, dass im implantierten Zustand des expandierten medizinischen Instruments zum einen eine maximale Expansion der Herzklappenprothese und zum anderen eine optimale seitliche Abdichtung zur Gefäßwand erzielt wird.

Insbesondere ist es von Vorteil, wenn der aus dem flexiblen Geflecht, insbesondere Drahtgeflecht gebildete Stent mit einer im Mittenbeich angeordneten selbst-expandierbaren Herzklappenprothese ausgelegt ist, dass der Stent mit der Herzklappenprothese zu jedem Zeitpunkt während der Implantation des medizinischen Instruments wieder in das Kathetersystem rückziehbar und aus dem Körper des Patienten entfernbar ist.

Im Hinblick auf das flexible Geflecht ist vorgesehen, dass dieses aus Nitinol oder aus einem anderen Material mit Formgedächnis- oder Memory-Effekt bestehen kann. Als andere Materialien könnten beispielsweise Kupfer-Zink-Aluminium-Legierungen, Gold-Kadmium-Legierungen oder auch Legierungen auf einer Eisen-Basis, wie zum Beispiel Eisen-Mangan, Silizium-Legierungen, aber auch Kunststoffe in Frage kommen, die allesamt dadurch gekennzeichnet sind, dass sie über ein extrem hoher Erinnerungsvermögen verfügen.

Schließlich ist im Hinblick auf die Verwendung des medizinischen Instruments besonders bevorzugt vorgesehen, dass der aus dem flexiblen Geflecht bestehende Stent mit der in seinem Mittenbereich befindlichen Herzklappenprothese zum Ersatz sowohl der Aortenals auch der Mitral-, Pulmonal- oder Trikuspidalklappe verwendet werden kann.

Im folgenden wird die Erfindung unter Bezugnahme auf die beigefügten Zeichnungen näher erläutert, wobei in den Zeichnungen folgendes gilt:
- Fig. 1: zeigt eine bevorzugte Ausführungsform des erfindungsgemäßen medizinischen Instruments während des Einführens in den Körper des Patienten, wobei das flexible Geflecht, welches hier den Aortenklappenstent ausbildet, seine erste vorab festgelegte Formgebung aufweist;
- Fig. 2: zeigt das medizinische Instrument gemäß Fig. 1 in einem ersten Zustand, in welchem der Aortenklappenstent aus dem Einführ-Kathetersystem freigesetzt wird;
- Fig. 3: zeigt das medizinische Instrument gemäß Fig. 2 in einem fortgeschrittenen zweiten Zustand während des Freisetzens des Aortenklappenstents aus dem Einführ-Kathetersystem;
- Fig. 4: zeigt das medizinische Instrument gemäß Fig. 3 in einem noch weiter fortgeschrittenen dritten Zustand während des Freisetzens des Aortenklappenstents aus dem Einführ-Kathetersystem;
- Fig. 5: zeigt einen Zustand, bei welchem der Aortenklappenstent und somit das medizinischen Instrument gemäß den Figuren 1 bis 4 vollständig expandiert und in der Höhe der defekten Herzklappe des Patienten implantiert ist;
- Fig. 6: zeigt eine perspektivische Ansicht des expandierten medizinischen Instruments gemäß der bevorzugten Ausführungsform;
- Fig. 7: zeigt einen möglichen Implantationsweg für das medizinische Instrument gemäß der bevorzugten Ausführungsform.

Bei der in den Zeichnungen dargestellten Ausführungsform des erfindungsgemäßen selbstexpandierbaren medizinischen Instruments 100 zur Behandlung von Defekten am Herzen eines Patienten, handelt es sich um ein selbstexpandierbares medizinisches Instrument zur transvaskulären Implantation einer Herzklappenprothese 30, wobei das medizinische Instrument 100 mittels eines Kathetersystems 40 minimal-invasiv in den Körper eines Patienten einführbar ist und einen Stent 1 aus einem flexiblen Geflecht (2) dünner Drähte oder Fäden 2' aufweist.

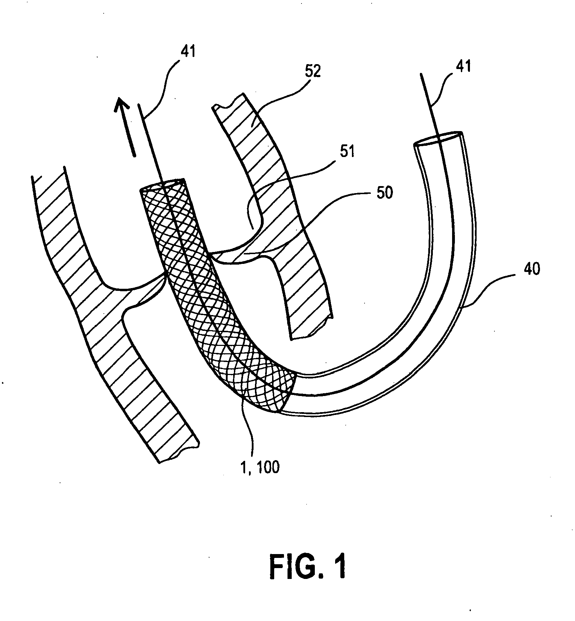

Wie in Fig. 1 gezeigt, liegt der aus dem flexiblen Geflecht 2 gebildete Stent 1 während des Einführens des medizinischen Instruments 100 in den Körper des Patienten in einer ersten vorab festgelegten Formgebung vor. Der Stent 1 weist in seinem Mittenbereich 15 ferner eine selbst-expandierbare Herzklappenprothese 30 auf, die in Fig. 1 von dem Geflecht 2 verdeckt und somit nicht explizit dargestellt ist. Wie es später beschrieben wird, entfaltet sich die selbst-expandierbare Herzklappenprothese 30 bei der Freisetzung des medizinischen Instruments 100 bzw. des Stents 1 aus dem Kathetersystem 40 in einer selbstständigen Weise.

Der Fig. 1 ist insbesondere zu entnehmen, dass sich der aus dem Geflecht 2 gebildete Stent 1 mit der im Mittenbereich 15 angeordneten (in Fig. 1 nicht explizit gezeigten) Herzklappenprothese 30 auf den Durchmesser des bei dem transvaskulären Operationseingriff verwendeten Kathetersystems 40 verjüngen lässt. In diesem Zustand liegt das medizinische Instrument 100 in seinem zusammengefalteten Zustand vor.

Im einzelnen ist in Fig. 1 ist ein Zustand gezeigt, unmittelbar bevor das medizinische Instrument 100 in seinem zusammengefalteten Zustand mit Hilfe eines Führungsdrahtes 41 durch die defekte Aortenklappe 50 des Patienten in Richtung der Aorta ascendens vorgebracht wird, und nachdem das medizinische Instrument 100 transseptal mit Hilfe eines Einführ-Kathetersystems 40 in den linken Ventrikel eingeführt wurde. Wie bereits angedeutet, ist in der in Fig. 1 gezeigten Darstellung von dem medizinischen Instrument 100 lediglich das flexible Geflecht 2 zu erkennen, welches die Funktion des Aortenklappenstents 1 übernimmt, und in dessen Mittenbereich 15 die (nicht explizit dargestellte) zusammengefaltete Herzklappenprothese 50 angeordnet ist.

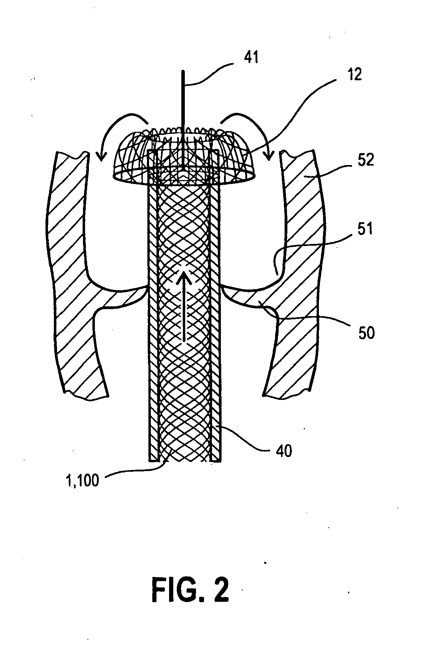

In Fig. 2 ist ein Zustand gezeigt, bei welchem - ausgehend von der in Fig. 1 gezeigten Position - der erste Teil des inneren Drahtgeflechtes 2 des Stents 1 aus dem entsprechenden Einführkathetersystem 40 herausgestülpt wurde, wobei sich dieser Teil, der im vollständig expandierten Zustand des medizinischen Instruments 100 den am distalen Retentionsbereich 10 des Stents vorgesehenen Wulstabschnitt 12 ausbildet, pilzförmig nach lateral wölbt. Im implantierten Zustand des medizinischen Instruments 100 greift der lateral nach außen gestülpten Wulstabschnitt 12 in zumindest eine Tasche 51 der defekten Herzklappe 50 des Patienten ein, wie es nachfolgend im einzelnen beschrieben wird.

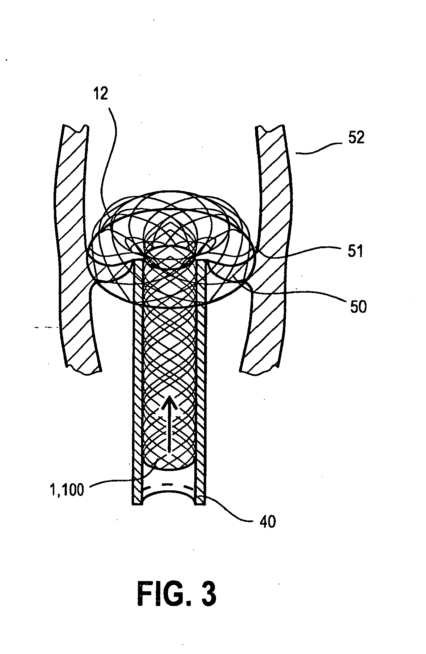

Weiter ist in Fig. 3 ein Zustand gezeigt, in welchem der gesamte Stent 1 bis auf die Höhe der defekten Aortenklappe 50 zurückgezogen ist, wo die defekte Klappe 50 formschlüssig in der "Taille" des Stents 1, d.h. im Mittenbereich 15 des nach der vollständigen Entfaltung doppelpilzförmigen Stents 1 eingehakt wird, nachdem der distale (obere) Teil des Drahtgeflechts 2 ausgestülpt und der Wulstabschnitt 12 fertig ausgebildet wurde.

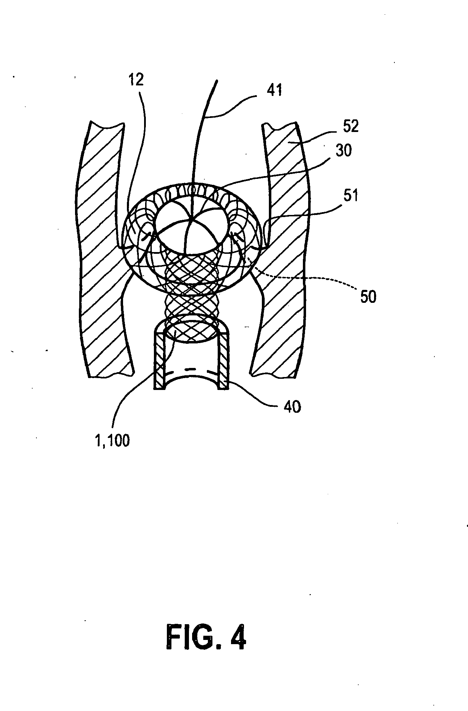

Andererseits ist in Fig. 4 ein Zustand gezeigt, bei welchem durch weiteres Ausfahren des aus dem Geflecht 2 gebildeten Stents 1 aus dem Kathetersystem 40 die im Inneren des Stents 1 in seinem Mittenbereich 15 vorgesehene selbstexpandierbare Aortenklappenprothese 30 hervortritt. In diesem Zustand steht die defekte (alte) Klappe 50 des Patienten mit dem taillierten Mittenbereich 15 des Stents 1 in Eingriff. Ferner ist der Wulstabschnitt 12 am distalen Retentionsbereich 10 des Stents 1 durch pilzförmiges Umstülpen des distalen Endes des Geflechts 2 nach außen ausgebildet ist, wobei der Wulstabschnitt 12 in die Taschen 51 der defekten Herzklappe 50 des Patienten gestülpt sind, so dass dieser als Mittel zum Positionieren des medizinischen Instruments 100 auf Höhe der defekten Herzklappe 50 des Patienten dient.

Bei einem weiteren Ausfahren des Stents 1 aus dem Kathetersystem 40 entfaltet sich schließlich der proximale Retentionsbereich 20 des Stents 1, wobei dieser dann aufgrund der selbstexpandierbaren Eigenschaft des Geflechts 2 eine kraftschlüssige Verbindung mit der Gefäßwand 52 in unmittelbarer Nähe der defekten Herzklappe 50 bildet. Gleichzeitig wird der Mittenbereich 15 des Stents 1 an die Aortenwand 52 gedrückt, wobei das selbstexpandierbare Geflecht 2 weiter expandiert und dabei die Klappenprothese 30 aufspannt.

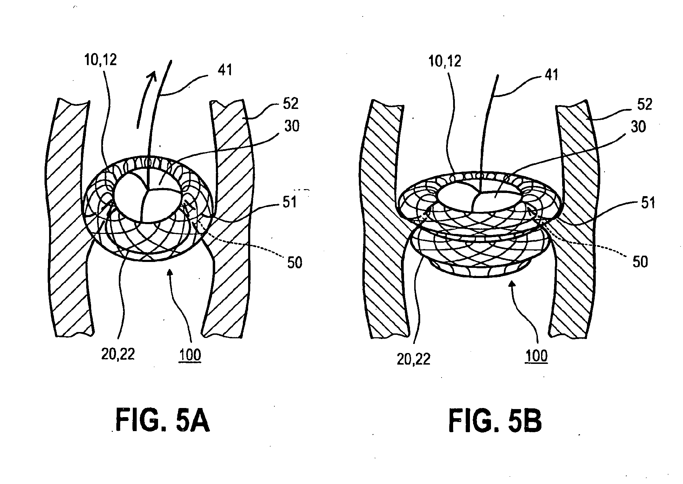

In Fig. 5a und Fig. 5b ist jeweils ein Zustand gezeigt, bei welchem die mechanische Herzklappenprothese 30 richtig positioniert ist und vollständig schließt, wobei die defekte (alte) Klappe 50 im Herzen verbleibt und gegen die Gefäßwand 52 gepresst wird. Des weiteren ist angedeutet, dass der Führungsdraht 41 nach eine Überprüfung des richtigen Sitz und der fehlerfreien Klappenfunktion der mechanischen Herzklappenprothese 30 wieder entfernt werden kann. Dabei sei darauf hingewiesen, dass bei Klappenfehlfunktion der Stent 1 mit der integrierten Herzklappenprothese 30 mit Hilfe des Führungsdrahtes 41 gegebenenfalls wieder in das Einführ-Kathetersystem zurückgezogen und durch einen anderen Stent mit integrierter Herzklappenprothese ersetzt werden kann.

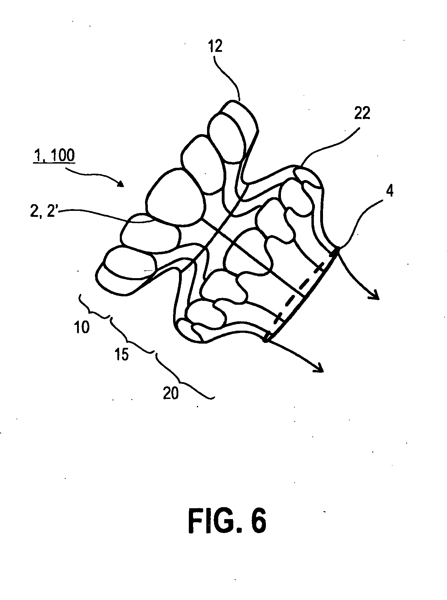

In Fig. 6 ist in einer perspektivischen Ansicht das expandierte medizinische Instrument 100 gemäß der bevorzugten Ausführungsform gezeigt. Es ist zu erkennen, dass der Stent 1 in dem expandierten Zustand des medizinischen Instruments 100 eine hantelähnliche Formgebung aufweist, wobei der aus dem flexiblen Geflecht 2 gebildete Stent 1 mit der (in Fig. 6 nicht erkennbaren) im Mittenbereich 15 innerhalb des Stents 1 angeordneten selbst-expandierbaren Herzklappenprothese 30 ausgelegt ist, dass die zweite Formgebung des Stents 1 an den anatomischen Bedingungen so anpassbar ist, dass im implantierten Zustand des expandierten medizinischen Instruments 100 zum einen eine maximale Expansion der Herzklappenprothese 30 und zum anderen eine optimale seitliche Abdichtung zur Gefäßwand 52 erzielbar ist.

Ferner ist der Fig. 6 zu entnehmen, dass bei dem expandierten medizinischen Instrument 100 der Mittenbereich 15 des Stents 1 einen im Vergleich zum proximalen und distalen Retentionsbereich 10 und 20 kleineren Durchmesser aufweist, wobei der Mittenbereich 15 eine Länge aufweist, die in etwa der Länge der defekten Herzklappe 50 entspricht.

Darüber hinaus ist bei der dargestellten Ausführungsform des medizinischen Instruments 100 vorgesehen, dass der Stent 1 hier an seinem proximalen Ende eine mit einem nicht gezeigten Explantations-Kathetersystem in Eingriff bringbare Fassung 4 in Gestalt eines Ringes aufweist, wobei das medizinische Instrument 100 ausgelegt ist, dass es sich durch externe Manipulation aus seinem expandierten Zustand in seinen zusammengefalteten Zustand bringen lässt.

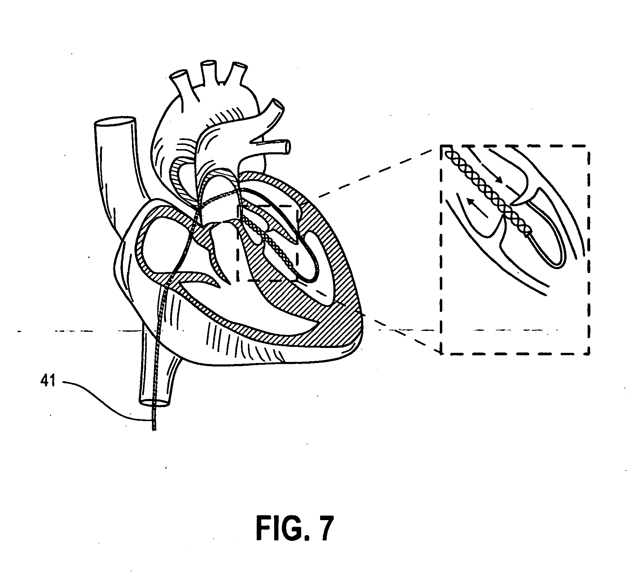

In Fig. 7 ist gezeigt, wie ein Führungsdraht 41 über die Hohlvene bis zum rechten Vorhof über das Vorhofseptum in den linken Vorhof und weiter in die linke Herzkammer und von dort über den linkventrikulären Ausflusstrakt und die Aortenklappe bis in die Aortaassendenz vorgeschoben wird.

Besonders bevorzugt ist, dass zum transvaskulären Ersatz von beispielsweise einer defekten Aortenklappe 50 eines Patienten in das Zentrum im Mittenbereich 15 des aus dem flexiblen Geflecht 2 gebildeten Stents 1 eine geeignete Klappenprothese 30 eingenäht oder andersartig befestigt wird. Der Stent 1 kann dann mit der integrierten Klappenprothese 30 auf den Durchmesser eines bei dem transvaskulären Operationseingriff verwendeten Kathetersystems 40 verjüngt werden und über das Einführ-Kathetersystem entweder vom Venensystem aus, nach Passage des Vorhofseptums, vom rechten Vorhof in den linken Vorhof und von dort weiter in den linken Ventrikel und den links-ventrikulären Ausflusstrakt vorgebracht werden.

Auf Höhe der defekten (alten) Aortenklappe 50 wird der aus dem Geflecht 2 ausgebildete Stent 1 aus dem Einführ-Kathetersystem 40 freigesetzt, wie es in Fig. 1 gezeigt ist. Durch das stufenweise Freisetzen des Stents 1 wird zunächst nur der distale Retentionsbereich 10 derart freigegeben, dass er sich pilzförmig nach Außen umstülpt, wie es in Fig. 2 bis Fig. 4 angedeutet ist. Anschließend kann durch ein vorsichtiges Führen des medizinischen Instruments 100 in Richtung Ventrikel der Wulstabschnitt 12 in die Taschen 51 der defekten alten Klappe 50 des Patienten formschlüssig vorgebracht werden. Die mittlere Taille bzw. der Mittenbereich 15 des Stents 1, in der sich die Klappenprothese 30 befindet, sitzt nun formschlüssig auf Höhe der alten Herzklappe (50), wie es in Fig. 4 angezeigt ist.

Anschließend wird auch der proximale Retentionsbereich 20 des Stents 1 aus dem Kathetersystem 40 ausgestoßen, wobei sich die künstliche Klappenprothese 30 entfaltet und gleichzeitig die alte, defekte Herzklappe 50 aufgrund der selbst expandierenden Eigenschaften des Drahtgeflechtes 2 gegen die Wand 52 gepresst wird (vgl. Fig. 5). In diesem Zustand stülpt sich der Wulstabschnitt bzw. Krempenbereich 12 am distalen Retentionsbereich 10 invers im links-ventrikulären Ausflusstrakt nach außen und bewirkt so eine zusätzliche mechanische Abstützung des medizinischen Instruments und sichere Verankerung. Mit Hilfe des am Stent 1 bis dahin noch verankerten Führungsdrahtes 41 kann bei Fehlfunktion der Stent 1 mit der integrierten Klappenprothese 30 ggf. wieder entfernt werden, wie es in Fig. 5a und Fig. 5b angedeutet ist.

Der Implantationsweg des doppelpilzförmigen Herzklappenstents 1 muss nicht zwingend transvenös und über das Vorhofseptum erfolgen. Ebenso ist es mit einem Kathetersystem 40 möglich, dieses Implantationsverfahren retrograd über den Aortenbogen in der oben beschriebenen Weise durchzuführen. Ferner bietet der in dieser Weise konstruierte Herzklappenstent 1 mit seiner vorgegebenen medialen Taillierung 15 die Möglichkeit, über eine integrierte Fassung (Ring) der Stent 1 mit der integrierten Herzklappenprothese 30 nachträglich wieder zu fassen und durch Längsextension des Drahtgeflechtes derart zusammenzufalten, dass es über einen Katheterschlauch wieder entfernt werden kann.

Der gesamte Implantationsweg ist detailliert in Abbildung 7 dargelegt. Zunächst wird ein Führungsdraht 41 über die Hohlvene zum rechten Vorhof und durch das Vorhofseptum in den linken Vorhof eingebracht. Vom linken Vorhof wird der Führungsdraht durch die linke Herzkammer, den links-ventrikulären Ausflusstrakt in die Aorta geschoben (Fig. 7). Über die Leitschiene des Führungsdrahtes 41 wird nun der Einführkatheter 40 bis in den links-ventrikulären Ausflusstrakt und die Aortenklappenebene vorgeschoben. Hier erfolgt dann die oben beschriebene Klappenimplantation.

Alternativ kann ein Führungsdraht 41 retrograd vom Aortenbogen kommend durch die Aortenklappe in den linken Ventrikel geschoben werden. Hier ist bei modifiziertem Katheterschlauch eine analoge Implantation des Aortenklappenstents 1 wie zuvor angedeutet möglich.

Durch eine bauartgegebene Integration von Halteelementen am selbstexpandierbaren Stent 1 kann dieser auch nach erfolgter Implantation mittels eines Spezialkatheters wieder explantiert werden. Dazu sollte der distale oder proximale Retentionsbereich 10, 20 des Stents 1 an mehreren, vorzugsweise an mehr als drei Haltepunktionen durch Führungsdrähte 41 in einen Katheter 40 hineingezogen. Dabei wird, umgekehrt der Implantation, der pilzförmige proximale Wulstabschnitt 22 am proximalen Retentionsbereich 20 des Stents 1 zurückgeschlagen, wodurch sich das Drahtgeflecht 2 wieder streckt und einen Zustand annimmt, wie er in Fig. 4 gezeigt ist. Anschließend kann der Eingriff bzw. Verankerung zwischen dem Wulstabschnitt 12 am distalen Retentionsbereich 10 des Stents 1 und den Taschen 51 der körpereigenen defekten Herzklappe gelöst werden.

Der aus dem flexiblen Drahtgeflecht 2 bestehende und sich dem Klappenring und ggf. Ausflusstrakt des menschlichen Herzens anpassende Stent 1 mit einer im seinem Mittenbereich 15 integrierten Klappenprothese 30 kann in gleicher Weise für den Ersatz der Mitralklappe sowie für einen Ersatz der Pulmonal- oder Trikuspidalklappe genutzt werden.

Es ist ersichtlich, dass sich die erfindungsgemäße Lösung gegenüber bisher bekannten medizinischen Instrumenten zum transvaskulären Ersatz von erkrankten Herzklappen insbesondere durch die folgende Merkmale auszeichnet:
1. Mit dem aus dem selbstexpandierbaren Geflecht gebildeten Stent, in dessen Mitte sich eine Klappenprothese befindet, wird die alte, erkrankte Herzklappe umfasst, eingestülpt und gegen die Gefäßwand gepresst.
2. Die Klappenprothese im Stent kann sowohl antegrad (mittels transseptaler Punktion) als auch retrograd implantiert werden.
3. Der Stent bzw. das flexible Geflecht passt sich den anatomischen Gegebenheiten des Klappenringes und des Ausflusstraktes des Herzens optimal an, wodurch eine verbesserte seitliche Abdichtung bei dem implantierten medizinischen Instrument erzielt wird.
4. Bei einer Fehlfunktion der Klappenprothese kann der Stent mit der integrierten Klappenprothese wieder in das Einführ-Kathetersystem zurückgezogen und vollständig aus dem Körper des Patienten entfernt werden.
5. Durch die hohe Flexibilität des Drahtgeflechtes ist im Gegensatz zu herkömmlichen Stentklappen eine Implantation auch bei sehr gewinkeltem Zugangsweg möglich.
6. Das selbstexpandierende Drahtgeflecht kann zum Ersatz sowohl der Klappen der linken als auch der rechten Herzkammer und zum Ersatz sowohl der Segelals auch der Taschenklappen des Herzens verwendet werden, da es sich flexibel den anatomischen Gegebenheiten anpasst und um die alte erkrankte Klappe legt.
7. Durch das Einstülpen der alten, erkrankten Klappe in das selbstexpandierende Drahtgeflecht wird eine Embolisation von Teilen der alten Klappe verhindert.
8. Durch eine bauartgegebene Integration von Halteelementen am selbstexpandierenden Drahtgeflecht kann diese auch nach erfolgter Implantation mittels eines Spezialkatheters explantiert werden.

Es sei darauf hingewiesen, dass die Ausführung der Erfindung nicht auf das unter Bezugnahme der Figuren 1 bis 7 beschriebene Ausführungsbeispiele beschränkt ist, sondern auch in einer Vielzahl von Varianten möglich ist.

### Bezugszeichenliste

- 1: Stent
- 2: Geflecht
- 2': Fäden/Draht des Geflechts
- 4: Fassung
- 10: distaler Retentionsbereich des Stents
- 12: Wulstabschnitt am distalen Retentionsbereich
- 15: Mittenbereich des Stents
- 20: proximaler Retentionsbereich des Stents
- 22: Wulstabschnitt am proximalen Retentionsbereich
- 30: Klappenprothese
- 40: Kathetersystem
- 41: Führungsdraht
- 50: körpereigene Herzklappe
- 51: Tasche der körpereigenen Herzklappe
- 52: Gefäßwand
- 100: medizinisches Instrument

## Patentansprüche

1. Selbstexpandierbares medizinisches Instrument (100) zur Behandlung von Defekten am Herzen eines Patienten, insbesondere zur transvaskulären Implantation einer Herzklappenprothese (30), wobei das medizinische Instrument (100) mittels eines Kathetersystems (40) minimal-invasiv in den Körper eines Patienten einführbar ist und einen Stent (1) aus einem flexiblen Geflecht (2) dünner Drähte
oder Fäden (2') aufweist, wobei der aus dem flexiblen Geflecht (2) gebildete Stent (1) während des Einführens des medizinischen Instruments (100) in den Körper des Patienten eine erste vorab festlegbare Formgebung und im implantierten Zustand des medizinischen Instruments (100) eine zweite vorab festlegbare Formgebung aufweist, wobei das medizinische Instrument (100) in der ersten Formgebung des aus dem flexiblen Geflecht (2) bestehenden Stents (1) in einem zusammengefalteten Zustand und in der zweiten Formgebung des aus dem flexiblen Geflecht (2) bestehenden Stents (1) in einem expandierten Zustand vorliegt, und wobei der aus dem flexiblen Geflecht (2) bestehende Stent (1) in seiner zweiten vorab festlegbaren Formgebung und in dem expandierten Zustand des medizinische Instruments (100) folgendes aufweist:
- einen distalen Retentionsbereich (10);
- einen proximalen Retentionsbereich (20); und
- einen zwischen dem distalen und proximalen Retentionsbereich (10, 20) liegenden Mittenbereich (15),
wobei der Stent (1) des expandierten medizinischen Instruments (100) am Mittenbereich (15) einen kleineren Durchmesser als am proximalen und/oder distalen Retentionsbereich (10, 20) aufweist, und wobei der Mittenbereich (15) ausgelegt ist, im implantierten Zustand des medizinischen Instruments (100) auf Höhe der defekten Herzklappe (50) eine formschlüssige Verbindung mit der Gefäßwand (52) zu bilden,
**dadurch gekennzeichnet, dass**
der distale Retentionsbereich (10) des Stents (1) in dem expandierten Zustand des medizinischen Instruments (100) einen lateral nach außen umgestülpten Wulstabschnitt (12) aufweist, welcher im implantierten Zustand des medizinischen Instruments (100) mit zumindest einer Tasche (51) der defekten Herzklappe (50) des Patienten in Eingriff bringbar ist.

2. Medizinisches selbstexpandierbares Instrument (100) nach Anspruch 1,
wobei der Stent (1) ferner eine im Mittenbereich (15) angeordnete selbst-expandierbare Herzklappenprothese (30) aufweist, die sich bei der Freisetzung des medizinischen Instruments (100) aus dem Kathetersystem (40) selbstständig entfaltet.

3. Medizinisches Instrument (100) nach Anspruch 1 oder 2,
wobei das Geflecht (2) ein Schlauchgeflecht ist, und wobei das medizinische Instrument (100) in seinem expandierten Zustand eine zum proximalen und distalen Ende hin offene Form aufweist.

4. Medizinisches Instrument (100) nach einem der vorhergehenden Ansprüche,
wobei bei dem expandierten medizinischen Instrument (100) der Wulstabschnitt (12) am distalen Retentionsbereich (10) des Stents (1) durch pilzförmiges Umstülpen des distalen Endes des Geflechts (2) nach außen gebildet ist.

5. Medizinisches Instrument (100) nach Anspruch 4,
wobei im implantierten Zustand des expandierten medizinischen Instruments (100) der Wulstabschnitt (12) am distalen Retentionsbereich (10) des Stents (1) in die zumindest eine Tasche (51) der defekten Herzklappe (50) des Patienten stülpbar ist und somit als Mittel zum Positionieren des medizinischen Instruments (100) auf Höhe der defekten Herzklappe (50) des Patienten dient.

6. Medizinisches Instrument (100) nach einem der vorhergehenden Ansprüche, wobei bei dem expandierten medizinischen Instrument (100) der proximale Retentionsbereich (20) des Stents (1) ausgelegt ist, im implantierten Zustand des expandierten medizinischen Instruments (100) aufgrund der selbstexpandierbaren Eigenschaft des Geflechts (2) eine kraftschlüssige Verbindung mit der Gefäßwand (52) in unmittelbarer Nähe der defekten Herzklappe (50) zu bilden.

7. Medizinisches Instrument (100) nach einem der vorhergehenden Ansprüche, wobei bei dem expandierten medizinischen Instrument (100) der Mittenbereich (15) des Stents (1) ausgelegt ist, im implantierten Zustand des medizinischen Instruments (100) aufgrund der selbstexpandierbaren Eigenschaft des Geflechts (2) die defekte Herzklappe (50) des Patienten gegen die an der distalen Seite der defekten Herzklappe (50) liegende Gefäßwand (52) zu drücken.

8. Medizinisches Instrument (100) nach einem der vorhergehenden Ansprüche, wobei der Stent (1) in dem expandierten Zustand des medizinischen Instruments (100) eine hantelähnliche Formgebung aufweist.

9. Medizinisches Instrument (100) nach einem der vorhergehenden Ansprüche, wobei bei dem expandierten medizinischen Instrument (100) der Mittenbereich (15) des Stents (1) einen im Vergleich zum proximalen und distalen Retentionsbereich (10, 20) kleineren Durchmesser aufweist, und wobei der Mittenbereich (15) eine Länge aufweist, die in etwa der Länge der defekten Herzklappe (50) entspricht.

10. Medizinisches Instrument (100) nach einem der vorhergehenden Ansprüche, wobei sich der aus dem Geflecht (2) gebildete Stent (1) mit einer im Mittenbereich (15) angeordneten selbst-expandierbaren Herzklappenprothese (30) auf den Durchmesser eines bei dem transvaskulären Operationseingriff verwendeten Kathetersystems (40) verjüngen lässt.

11. Medizinisches Instrument (100) nach einem der vorhergehenden Ansprüche, wobei der Stent (1) an seinem proximalen und/oder distalen Ende eine mit einem Explantations-Kathetersystem (40) in Eingriff bringbare Fassung (4) aufweist, und wobei das medizinische Instrument (100) ausgelegt ist, dass es sich durch externe Manipulation aus seinem expandierten Zustand in seinen zusammengefalteten Zustand bringen lässt.

12. Medizinisches Instrument (100) nach einem der vorhergehenden Ansprüche, wobei im implantierten Zustand des expandierten medizinischen Instruments (100) das flexible Geflecht (2), insbesondere Drahtgeflecht, des Stents (1) in mehrschichtiger Anordnung um die defekte Herzklappe (50) herum liegt.

13. Medizinisches Instrument (100) nach einem der vorhergehenden Ansprüche, wobei der aus dem flexiblen Geflecht (2), insbesondere Drahtgeflecht gebildete Stent (1) mit einer im Mittenbereich (15) angeordneten selbst-expandierbaren Herzklappenprothese (30) ausgelegt ist, dass die zweite Formgebung des Stents (1) an den anatomischen Bedingungen so anpassbar ist, dass im implantierten Zustand des expandierten medizinischen Instruments (100) zum einen eine maximale Expansion der Herzklappenprothese (30) und zum anderen eine optimale seitliche Abdichtung zur Gefäßwand (52) erzielbar ist.

14. Medizinisches Instrument (100) nach einem der vorhergehenden Ansprüche, wobei der aus dem flexiblen Geflecht (2), insbesondere Drahtgeflecht gebildete Stent (1) mit einer im Mittenbereich (15) angeordneten selbst-expandierbaren Herzklappenprothese (30) ausgelegt ist, dass der Stent (1) mit der Herzklappenprothese (30) zu jedem Zeitpunkt während der Implantation des medizinischen Instruments (100) wieder in das Kathetersystem (40) rückziehbar und aus dem Körper des Patienten entfernbar ist.

15. Medizinisches Instrument (100) nach einem der vorhergehenden Ansprüche, wobei der aus dem flexiblen Geflecht (2), insbesondere Drahtgeflecht gebildete Stent (1) mit einer im Mittenbereich (15) angeordneten selbst-expandierbaren Herzklappenprothese (30) ausgelegt ist, dass der Stent (1) mit der Herzklappenprothese (30) nach erfolgter Freisetzung mit Hilfe eines Kathetersystems (40) und Führungsdrähten (41) wieder rückziehbar und explantierbar ist.

16. Medizinisches Instrument (100) nach einem der vorhergehenden Ansprüche, wobei das flexible Geflecht (2) aus einem Material mit Memory-Effekt, insbesondere Nitinol oder Memory Kunststoffen besteht.

17. Medizinisches Instrument (100) nach einem der vorhergehenden Ansprüche, wobei der aus dem flexiblen Geflecht (2), insbesondere Drahtgeflecht gebildete Stent (1) mit einer im Mittenbereich (15) angeordneten selbst-expandierbaren Herzklappenprothese (30) ausgelegt ist, die Aortenklappe, die Mitralklappe, die Pulmonalklappe oder die Trikuspidalklappe eines Patienten zu ersetzen.

## Claims

1. A self-expandable medical instrument (100) for treating defects in a patient's heart, in particular for the transvascular implantation of a prosthetic heart valve (30), wherein the medical instrument (100) can be introduced into the body of a patient in a minimally-invasive procedure using a catheter system (40) and comprises a stent (1) made from a flexible mesh (2) of thin wires or filaments (2'), wherein the stent (1) configured from flexible mesh (2) exhibits a first predefinable shape during the insertion of the medical instrument (100) into the patient's body and a second predefinable shape in the implanted state of the medical instrument (100), wherein said medical instrument (100) is in a collapsed state in the first shape of the stent (1) composed of the flexible mesh (2) and in an expanded state in the second shape of the stent (1) composed of the flexible mesh (2), and wherein in its second predefinable shape and in the expanded state of the medical instrument (100), the stent (1) composed of the flexible mesh (2) exhibits the following:
- a distal retention area (10);
- a proximal retention area (20); and
- a center area (15) positioned between the distal and the proximal retention areas (10, 20),
wherein the center area (15) of the stent (1) of the expanded medical instrument (100) exhibits a smaller diameter than its proximal and/or distal retention areas (10, 20), and wherein the center area (15) is configured to realize a form-fit connection with the vascular wall (52) at the height of the defective heart valve (50) in the implanted state of the medical instrument (100),
**characterized in that**
the distal retention area (10) of the stent (1) exhibits a laterally outward inverted beaded portion (12) in the expanded state of the medical instrument (100) which is engageable in at least one pocket (51) of the patient's defective heart valve (50) in the implanted state of the medical instrument (100).

2. The self-expandable medical instrument (100) according to claim 1,
wherein the stent (1) further exhibits a self-expandable prosthetic heart valve (30) disposed in its center area (15) which self-expands upon the medical instrument (100) being released from the catheter system (40).

3. The medical instrument (100) according to claim 1 or 2,
wherein the mesh (2) is a mesh tube, and wherein the medical instrument (100) exhibits a form open to the proximal and distal end in its expanded state.

4. The medical instrument (100) according to any one of the preceding claims,
wherein the beaded portion (12) at the distal retention area (10) of the stent (1) in the expanded medical instrument (100) is formed by the mushroom-shaped outward inversion of the distal end of the mesh (2).

5. The medical instrument (100) according to claim 4,
wherein the beaded portion (12) at the distal retention area (10) of the stent (1) in the expanded medical instrument (100) in the implanted state can be inverted into at least one pocket (51) of the patient's defective heart valve (50) and thus serves as a means for posi-tioning the medical instrument (100) at the height of the patient's defective heart valve (50).

6. The medical instrument (100) according to any one of the preceding claims,
wherein based on the self-expanding properties of the mesh (2), the proximal retention area (20) of the stent (1) in the expanded medical instrument (100) is configured so as to form a force-fit connection with the vascular wall (52) in the direct proximity of the defective heart valve (50) in the implanted state of the expanded medical instrument (100).

7. The medical instrument (100) according to any one of the preceding claims,
wherein based on the self-expanding properties of the mesh (2), the center area (15) of the stent in the expanded medical instrument (100) is configured so as to press the patient's defective heart valve (50) against the vascular wall (52) distal the defective heart valve (50) in the implanted state of the medical instrument (100).

8. The medical instrument (100) according to any one of the preceding claims,
wherein the stent (1) in the expanded state of the medical instrument (100) exhibits a shape similar to a barbell.

9. The medical instrument (100) according to any one of the preceding claims,
wherein the center area (15) of the stent (1) exhibits a smaller diameter in the expanded medical instrument (100) than its proximal and distal retention areas (10, 20), and wherein the center area (15) exhibits a length which corresponds approximately to the length of the defective heart valve (50).

10. The medical instrument (100) according to any one of the preceding claims,
wherein the stent (1) made of mesh (2) with a self-expandable prosthetic heart valve (2) arranged at its center area (15) tapers to the diameter of the catheter system (40) used in the transvascular surgical procedure.

11. The medical instrument (100) according to any one of the preceding claims,
wherein the stent (1) exhibits a mount (4) engageable with an explantation catheter system (40) on its proximal and/or distal end, and wherein the medical instrument (100) is configured such that an external manipulation will bring it from its expanded state into its collapsed state.

12. The medical instrument (100) according to any one of the preceding claims,
wherein in the implanted state of the expanded medical instrument (100), the flexible mesh (2), in particular wire mesh, of the stent (1) is disposed in a multi-layered arrangement around the defective heart valve (50).

13. The medical instrument (100) according to any one of the preceding claims,
wherein the stent (1) made from flexible mesh (2), in particular wire mesh, having a self-expandable prosthetic heart valve (30) arranged at its center area (15) is configured such that the second shape of the stent (1) is adaptable to the anatomical conditions so as to achieve a maximum expansion of the prosthetic heart valve (30) on the one hand and an optimum lateral sealing to the vascular wall (52) on the other in the implanted state of the expanded medical instrument (100).

14. The medical instrument (100) according to any one of the preceding claims,
wherein the stent (1) made from flexible mesh (2), in particular wire mesh, having a self-expandable prosthetic heart valve (30) arranged at its center area (15) is configured such that the stent (1) with the prosthetic heart valve (30) can be withdrawn back into the catheter system (40) and removed from the patient's body at any time during the implantation of the medical instrument (100).

15. The medical instrument (100) according to any one of the preceding claims,
wherein the stent (1) made from flexible mesh (2), in particular wire mesh, having a self-expandable prosthetic heart valve (30) arranged at its center area (15) is configured such that the stent (1) with the prosthetic heart valve (30) can be further retracted and explanted via a catheter system (40) and guide wires (41) following a successful release.

16. The medical instrument (100) according to any one of the preceding claims,
wherein the flexible mesh (2) is made from a material having memory effect, particularly nitinol or memory plastics.

17. The medical instrument (100) according to any one of the preceding claims,
wherein the stent (1) made from flexible mesh (2), in particular wire mesh, having a self-expandable prosthetic heart valve (30) arranged at its center area (15) is configured so as to replace a patient's aortic valve, mitral valve, pulmonary valve or tricuspid valve.

## Revendications

1. Instrument médical autoexpansible (100) pour le traitement de défauts du coeur d'un patient, en particulier pour l'implantation transvasculaire d'une prothèse de valvule cardiaque (30), dans lequel l'instrument médical (100) est susceptible d'être introduit dans le corps d'un patient au moyen d'un système à cathéter (40) de manière minimalement invasive, et comprend un stent (1) formé d'un treillis flexible (2) de fils ou de filaments minces (2'), dans lequel le stent (1) formé du treillis flexible (2) présente pendant l'introduction de l'instrument médical (100) dans le corps du patient une première conformation déterminable au préalable et présente dans l'état implanté de l'instrument médical (100) une seconde conformation déterminable au préalable, de sorte que l'instrument médical (100) se présente, dans la première conformation du stent (1) formé du treillis flexible (2), dans un état replié, et se présente, dans la seconde conformation du stent (1) formé du treillis flexible (2), dans un état en expansion, et dans lequel le stent (1) formé du treillis flexible (2) comprend, dans sa seconde conformation déterminable au préalable et dans l'état en expansion de l'instrument médical (100), les éléments suivants :
- une zone de rétention distale (10) ;
- une zone de rétention proximale (20) ; et
- une zone médiane (15) située entre la zone de rétention distale et la zone de rétention proximale (10, 20),
dans lequel le stent (1) de l'instrument médical en expansion (100) présente dans la zone médiane (15) un diamètre plus petit que dans la zone de rétention proximale et/ou dans la zone de rétention distale (10, 20), et dans lequel la zone médiane (15) est conçue, dans l'état implanté de l'instrument médical (100), pour former à la hauteur de la valvule cardiaque défectueuse (50) une liaison à coopération de formes avec la paroi du vaisseau (52),
**caractérisé en ce que**
la zone de rétention distale (10) du stent (1) présente, dans l'état en expansion de l'instrument médical (100), un tronçon de bourrelet (12) retroussé latéralement vers l'extérieur qui, dans l'état implanté de l'instrument médical (100), peut être amené en engagement avec au moins une poche (51) de la valvule cardiaque défectueuse (50) du patient.

2. Instrument médical autoexpansible (100) selon la revendication 1,
dans lequel le stent (1) comprend en outre une prothèse de valvule cardiaque (30) autoexpansible agencée dans la zone médiane (15), laquelle se déploie automatiquement lors du dégagement de l'instrument médical (100) hors du système à cathéter.

3. Instrument médical (100) selon la revendication 1 ou 2,
dans lequel le treillis (2) est un treillis en forme de tuyau, et dans lequel l'instrument médical (100) présente, dans son état en expansion, une forme ouverte vers l'extrémité proximale et vers l'extrémité distale.

4. Instrument médical (100) selon l'une des revendications précédentes,
dans lequel, dans l'instrument médical en expansion (100), le tronçon en bourrelet (12) au niveau de la zone de rétention distale (10) du stent (1) est formé par retroussement, à la manière d'un champignon, de l'extrémité distale du treillis (2) vers l'extérieur.

5. Instrument médical (100) selon la revendication 4,
dans lequel, dans l'état implanté de l'instrument médical en expansion (100), le tronçon en bourrelet (12) au niveau de la zone de rétention distale (10) du stent (1) peut être retroussé dans ladite au moins une poche (51) de la valvule cardiaque défectueuse (50) du patient, et servir ainsi de moyen pour le positionnement de l'instrument médical (100) à la hauteur de la valvule cardiaque défectueuse (50) du patient.

6. Instrument médical (100) selon l'une des revendications précédentes, dans lequel dans l'instrument médical en expansion (100) la zone de rétention proximale (20) du stent (1) est conçue pour, dans l'état implanté de l'instrument médical en expansion (100) et en raison de la propriété autoexpansible du treillis (2), établir une liaison à coopération de forces avec la paroi du vaisseau (52) au voisinage immédiat de la valvule cardiaque défectueuse (50).

7. Instrument médical (100) selon l'une des revendications précédentes, dans lequel dans l'instrument médical en expansion (100) la zone médiane (15) du stent (1) est conçue pour, dans l'état implanté de l'instrument médical (100) et en raison de la propriété autoexpansible du treillis (2), pousser contre la paroi du vaisseau (52) qui se trouve à l'extrémité distale de la valvule cardiaque défectueuse (50).

8. Instrument médical (100) selon l'une des revendications précédentes, dans lequel le stent (1) présente, dans l'état en expansion de l'instrument médical (100), une conformation semblable à une haltère.

9. Instrument médical (100) selon l'une des revendications précédentes, dans lequel dans l'instrument médical en expansion (100), la zone médiane (15) du stent (1) présente un diamètre plus petit par comparaison à la zone de rétention proximale et à la zone de rétention distale (10, 20), et la zone médiane (15) présente une longueur qui correspond approximativement à la longueur de la valvule cardiaque défectueuse (50).

10. Instrument médical (100) selon l'une des revendications précédentes, dans lequel le stent (1) formé du treillis (2) se laisse rétrécir, avec une prothèse de valvule cardiaque autoexpansible (30) agencée dans la zone médiane (15), au diamètre d'un système de cathéter (40) utilisé lors de l'opération transvasculaire.

11. Instrument médical (100) selon l'une des revendications précédentes, dans lequel le stent (1) comprend à son extrémité proximale et/ou à son extrémité distale une monture (4) qui peut être amenée en engagement avec un système de cathéter d'explantation (40), et dans lequel l'instrument médical (100) est conçu de telle manière qu'il peut être amené par une manipulation externe depuis son état en expansion jusqu'à son état replié.

12. Instrument médical (100) selon l'une des revendications précédentes, dans lequel dans l'état implanté de l'instrument médical en expansion (100), le treillis flexible (2), en particulier le treillis en fil, du stent (1) est disposé tout autour de la valvule cardiaque défectueuse (50) dans un agencement à plusieurs couches.

13. Instrument médical (100) selon l'une des revendications précédentes, dans lequel le stent (1) formé du treillis flexible (2), en particulier du treillis en fil, est conçu avec une prothèse de valvule cardiaque autoexpansible (30) agencée dans la zone médiane (15) de telle façon que la seconde conformation du stent (1) peut être ajustée aux conditions anatomiques de telle façon que dans l'état implanté de l'instrument médical en expansion (100) on peut atteindre d'une part une expansion maximum de la prothèse de valvule cardiaque (30) et d'autre part un étanchement latéral optimal vis-à-vis de la paroi du vaisseau (52).

14. Instrument médical (100) selon l'une des revendications précédentes, dans lequel le stent (1) formé du treillis flexible (2), en particulier du treillis en fil, est conçu avec une prothèse de valvule cardiaque autoexpansible (30) agencée dans la zone médiane (15) de telle façon que le stent (1) avec la prothèse de valvule cardiaque (30) est à tout instant à nouveau rétractable dans le système de cathéter (40) pendant l'implantation de l'instrument médical, et peut être enlevé hors du corps du patient.

15. Instrument médical (100) selon l'une des revendications précédentes, dans lequel le stent (1) formé du treillis flexible (2), en particulier du treillis en fil, est conçu avec une prothèse de valvule cardiaque autoexpansible (30) agencée dans la zone médiane (15) de telle façon que le stent (1) avec la prothèse de valvule cardiaque (30) peut être à nouveau rétractable et explanté, après sa libération, à l'aide d'un système de cathéter (14) et de fils de guidage (41).

16. Instrument médical (100) selon l'une des revendications précédentes, dans lequel le treillis flexible (2) est en un matériau présentant un effet à mémoire, en particulier du nitinol ou des matières plastiques à mémoire.

17. Instrument médical (100) selon l'une des revendications précédentes, dans lequel le stent (1) formé du treillis flexible (2), en particulier du treillis en fil, est conçu avec une prothèse de valvule cardiaque autoexpansible (30) agencée dans la zone médiane (15), pour remplacer la valvule de l'aorte, la valvule mitrale, la valvule pulmonaire ou la valvule tricuspide d'un patient.
